# EUROPÄISCHE PATENTSCHRIFT

(11) **EP 2 000 103 B1**
(45) Veröffentlichungstag und Bekanntmachung des Hinweises auf die Patenterteilung: **17.02.2016**
(21) Anmeldenummer: 08163808.2
(22) Anmeldetag: 11.07.2006
(51) Int. Cl.: A61B 17/122

(54) **Chirurgische Ligaturclips mit Träger**
Surgical ligature clips with carrier
Pinces de ligature chirurgicale avec porte-pinces

(30) Priorität: 11.01.2006 DE 202006000329 U
(43) Veröffentlichungstag der Anmeldung: 10.12.2008
(62) Teilanmeldung aus: 06762529.3
(73) Patentinhaber: Aesculap AG, 78532 Tuttlingen (DE)
(72) Erfinder: Lutze, Theodor, 78582, Balgheim (DE); Dworschak, Manfred, 78589, Dürbheim (DE)
(74) Vertreter: Hoeger, Stellrecht & Partner Patentanwälte mbB

(56) Entgegenhaltungen:
- EP-A- 0 567 965
- WO-A-98/18389
- US-A- 5 171 250
- US-A1- 2002 062 130
- "Titan Ligatur Clips und Anlegezangen" [Online] Februar 2002 (2002-02), AESCULAP , TUTTLINGEN DEUTSCHLAND , XP002400693 Gefunden im Internet: URL:http://www.mediwar.ch/d/produkte/chiru rgie/documents/TitanLigaturClips.pdf#searc h=%22titanligaturclips%20aesculap%22> [gefunden am 2006-09-26] * Prospektblatt C46811; Seiten 6-7 *

## Beschreibung

Die Erfindung betrifft einen Satz von mehreren chirurgischen Ligaturclips mit jeweils zwei Haltearmen, die an jeweils einem Ende über eine verformbare Verbindungsstelle miteinander verbunden sind und derart gegeneinander biegbar sind, dass die Arme aus einer Offenstellung, in der sie einen größeren Abstand voneinander haben, in eine Schließstellung gelangen, in der die einander zugewandten Innenseiten der Arme dauerhaft einander angenähert sind, wobei der Ligaturclip aus einem in sich geschlossenen Steg besteht, der im Bereich der beiden Arme und der Verbindungsstelle zwei nebeneinander liegende Abschnitte ausbildet, die an den der Verbindungsstelle abgewandten freien Enden der Arme ineinander übergehen.

Sowohl in der offenen als auch in der minimalinvasiven Chirurgie zählt die Applikation von Ligaturclips zum sicheren und schnellen Verschluss von Blutgefäßen und anderen Hohlorganen, wie z.B. von Gallengängen, zu einem bewährten Verfahren. Die Ligatur von Gefäßen und Hohlorganen ist dann erforderlich, wenn Gewebeanteile reseziert werden sollen und wenn bei der Durchtrennung von Gewebe eine Blutung entsteht, die mittels Koagulation nur schwer oder unsicher beherrscht werden kann.

Dabei kommen überwiegend Clips aus Reintitan zum Einsatz. Dies ist ein erprobter Werkstoff mit ausgezeichneten Biokompatibilitätseigenschaften. Es sind aber auch Clips aus resorbierbaren Materialien bekannt, dabei handelt es sich meist um Clips aus Polylactaten oder Clips aus anderen Kunststoffen, beispielsweise Polyetheretherketon.

Ligaturclips dieser Art werden beispielsweise vertrieben von der Firma AESCULAP AG & Co. KG unter dem Markennamen Challenger Ti (Prospektblätter der Firma AESCULAP AG & Co. KG, "Titan Ligatur Clips und Anlegezangen" C 468 11, Veröffentlichungsdatum September 2003; Challenger TiC461 11, Veröffentlichungsdatum Februar 2002).

Bekannte Clips dieser Art sind häufig V-förmig ausgebildet, die Breite der Clips variiert je nach Größe zwischen 0,5 und 1,2 Millimetern. Die Innenflächen sind in der Regel mit einer Profilierung versehen, um einen besseren Sitz auf dem Hohlorgan in Längsrichtung zu bewirken.

Trotz dieser Ausbildung besteht bei bekannten Clips die Gefahr, dass sie von dem geclipten Hohlorgan abgleiten. Üblicherweise werden die Hohlorgane nach dem Anlegen eines Ligaturclips dicht neben dem Clip durchtrennt, und dann kann es passieren, dass der Clip unbeabsichtigt seitlich von dem Hohlorgan heruntergeschoben wird, beispielsweise bei der Entnahme von Bauchtüchern, die über geclipten Gefäßen positioniert werden oder bei der minimalinvasiven Chirurgie durch Instrumente, die sich außerhalb des Sichtbereichs des Operateurs bewegen und daher von ihm nur schlecht kontrolliert werden können.

Aus der US-A 5,171,250, der WO-A-98/18389 und der EP-A-0 567 965 sind Ligaturclips bekannt, bei denen die beiden Arme zwei nebeneinander liegende Abschnitte ausbilden, die an den der Verbindungsstelle abgewandten freien Enden der Arme ineinander übergehen.

Man erhält auf diese Weise einen Doppelclip mit zwei nebeneinander liegenden Armteilen, durch diese doppelte Ausbildung der Armteile wird die Anlage des Ligaturclips am Hohlorgan deutlich verbessert. Man könnte einen solchen Ligaturclip auch betrachten als eine Baueinheit aus zwei nebeneinander liegenden Ligaturclips, die im Bereich des freien Endes der Arme durch eine Brücke miteinander verbunden sind. Die beiden Teile des Clips stabilisieren sich dadurch gegenseitig, so dass die Halteeigenschaften des am Hohlorgan angelegten Ligaturclips so gut sind, dass im allgemeinen darauf verzichtet werden kann, entsprechend der bisherigen Praxis an einem im Körper verbleibenden Hohlorgan unabhängig voneinander nebeneinander zwei derartige Ligaturclips zu platzieren.

In der US2002/0062130A1 ist ein Anlegeinstrument beschrieben, mit dem chirurgische Clips an Körpergewebe angelegt werden können. Bei diesem Instrment wird zum Anlegen eines Clips ein einzelner Clip auf das Instrument aufgeschoben und kann dann an der gewünschten Stelle des Körpers appliziert werden. Dieser Vorgang muss für jeden Clip wiederholt werden.

Es ist Aufgabe der Erfindung, die Handhabung einer größeren Anzahl von Ligaturclips beim Anlegen zu verbessern.

Diese Aufgabe wird bei einem Satz von Ligaturclips der eingangs beschriebenen Art erfindungsgemäß dadurch gelöst, dass der Satz einen gemeinsamen Träger für die Ligaturclips umfasst, der die parallel zueinander und in einer Reihe nebeneinander angeordneten Ligaturclips im Bereich des Zwischenraumes zwischen den nebeneinander liegenden Abschnitten durchsetzt.

Dadurch, dass die Ligaturclips einen von den Abschnitten des Steges umgebenen Zwischenraum aufweisen, kann dieser Zwischenraum genutzt werden, um einen Träger durch diesen hindurchzuschieben, so dass auf dem Träger eine größere Anzahl von gleich ausgebildeten Ligaturclips parallel zueinander nebeneinander aufgenommen werden können.

Dabei ist es insbesondere vorteilhaft, wenn die nebeneinander liegenden Abschnitte zumindest stellenweise dicht an dem Träger anliegen, dadurch ergibt sich ein Reibschluss zwischen den Ligaturclips und dem Träger, so dass diese auf dem Träger gegen unbeabsichtigte Verschiebung gesichert sind. Zur Abnahme eines Clips muss der Träger gegenüber dem Ligaturclip mit einer bestimmten Kraft verschoben werden, um diesen Kraftschluss zwischen Ligaturclip und Träger aufzuheben.

Bei einer weiteren bevorzugten Ausführungsform der Erfindung ist vorgesehen, dass die nebeneinander liegenden Abschnitte der Arme an ihrem der Verbindungsstelle abgewandten Ende in nach gegenüberliegenden Seiten seitlich abstehende, im wesentlichen quer zur Längsrichtung der nebeneinander liegenden Abschnitte verlaufende Transversalabschnitte einmünden. Es ergibt sich dadurch eine insgesamt T-förmige Ausgestaltung der Arme, wobei die Transversalabschnitte beispielsweise Klemmbacken ausbilden können.

Günstig ist es weiterhin, wenn die Transversalabschnitte eines Armes an ihren den nebeneinander liegenden Abschnitten abgewandten äußeren Enden in einen Klemmabschnitt übergehen, der im Abstand von den Transversalabschnitten auf deren den nebeneinander liegenden Abschnitten abgewandter Seite verläuft. Die Transversalabschnitte und der diese verbindende Klemmabschnitt bilden somit eine Klemmfläche am freien Ende der Arme aus, die nach beiden Seiten von den Armen absteht. Zwischen dem Klemmabschnitt einerseits und den Transversalabschnitten andererseits besteht ein Zwischenraum ähnlich wie zwischen den beiden nebeneinander liegenden Abschnitten der Arme, so dass in diesem Bereich geklemmtes Gewebe in den Zwischenraum eindringen kann. Dies führt zu einer besonders sicheren Anlage der aus Klemmabschnitt und den beiden Transversalabschnitten ausgebildeten Klemmfläche.

Insbesondere kann vorgesehen sein, dass der Klemmabschnitt im Wesentlichen parallel zu den Transversalabschnitten verläuft.

Die Transversalabschnitte können an den äußeren Enden bogenförmig in den Klemmabschnitt übergehen, insbesondere kreisbogenförmig. Es ergibt sich dadurch eine atraumatische Ausgestaltung der Klemmflächen im Bereich der äußeren Enden.

Vorzugsweise liegen die Transversalabschnitte und gegebenenfalls der Klemmabschnitt in derselben Ebene wie die an die Transversalabschnitte anschließenden nebeneinander liegende Abschnitte.

Der Klemmabschnitt kann geradlinig ausgebildet sein, gemäß einer besonders bevorzugten Ausführungsform verläuft er jedoch bogenförmig, insbesondere kreisbogenförmig.

Dabei ergibt sich eine besonders günstige Ausgestaltung, wenn der Klemmabschnitt von den nebeneinander liegenden Abschnitten wegweisend gebogen ist, wenn also die Mitte des Klemmabschnittes näher an den nebeneinander liegenden Abschnitten liegt als die äußeren Enden. Ein derartiger Clip kann besonders günstig an die Außenseite eines Hohlorgans angelegt werden, da der bogenförmige Verlauf des Klemmabschnittes dem gebogenen Außenverlauf des Hohlorgans folgen kann.

Gemäß einer bevorzugten Ausführungsform ist vorgesehen, dass mindestens ein Klemmabschnitt einen Vorsprung trägt, der bei aneinander liegenden Klemmabschnitten der beiden Arme in eine Ausnehmung des anderen Klemmabschnitt eintaucht. Dadurch werden einerseits die Klemmabschnitte beim Annähern relativ zueinander geführt, zum anderen durchdringen die Vorsprünge, die insbesondere als Stift ausgebildet sein können, das zwischen den Klemmabschnitten gehaltene Gewebe und fixieren dadurch Gewebe und Ligaturclip relativ zueinander.

An dem Klemmabschnitt eines Armes können über dessen Längsrichtung verteilt mehrere Vorsprünge und an dem Klemmabschnitt des anderen Armes entsprechende Ausnehmungen angeordnet sein, so dass eine Fixierung des Gewebes über die gesamte Länge des Klemmabschnittes erfolgt.

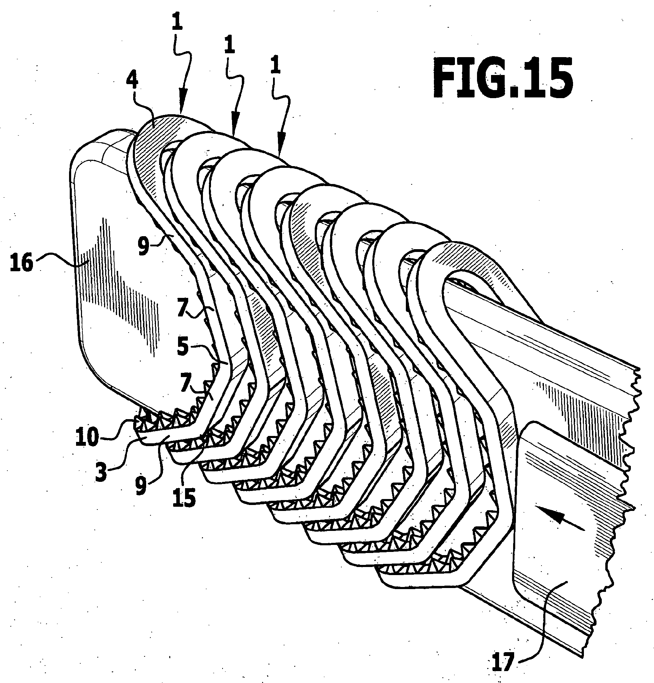

Die nachfolgende Beschreibung bevorzugter Ausführungsformen er Erfindung dient im Zusammenhang mit der Zeichnung der näheren Erläuterung. Ein erfindungsgemäßer Satz von Ligaturclips ist nur in Figur 15 dargestellt, die übrigen Figuren zeigen lediglich Ausführungsformen von Ligaturclips, die auf einem gemeinsamen Träger angeordnet werden und damit einen Teil des erfindungsgemäßen Satzes von Ligaturclips bilden können. Es zeigen:
- Figur 1:: einen Ligaturclip mit zwei nebeneinander liegenden Stegabschnitten in Offenstellung;
- Figur 2:: eine Teilansicht eines Anlegeinstrumentes mit einem Ligaturclip gemäß Figur 1 in Offenstellung des Ligaturclips;
- Figur 3:: eine perspektivische Teilansicht des Klemmbackens eines abgewandelten Ausführungsbeispiels eines Anlegeinstrumentes ohne Ligaturclip;
- Figur 4:: eine Ansicht ähnlich Figur 3 mit zwischen beiden Klemmbacken eingelegtem Ligaturclip vor dem Schließen des Ligaturclips;
- Figur 5:: einen Ligaturclip gemäß Figur 1 in der Anlage an einem Hohlorgan und in Schließstellung;
- Figur 6:: eine Ansicht ähnlich Figur 1 bei einem abgewandelten Ausführungsbeispiel eines Ligaturclips mit einer Mauszahnprofilierung an den freien Enden der beiden Arme;
- Figur 7: eine Ansicht ähnlich Figur 5 bei einem abgewandelten Ausführungsbeispiel mit einer weiteren Mauszahnprofilierung;
- Figur 8:: eine Ansicht eines Ligaturclips ähnlich Figur 1 mit einem Rastvorsprung an dem freien Ende eines Armes;
- Figur 9: eine Teilansicht eines abgewandelten Ausführungsbeispiels eines derartigen Rastvorsprunges vor dem Eintauchen in eine Rastausnehmung des anderen Armes des Ligaturclips;
- Figur 10:: eine Ansicht ähnlich Figur 7 mit einem hakenförmigen Rastelement an einem Arm des Ligaturclips;
- Figur 11a und Figur 11b:: eine Draufsicht bzw. eine Seitenansicht eines bandförmigen Materials vor dem Biegen zu einem Ligaturclip;
- Figur 12:: eine Seitenansicht des Ligaturclips der Figur 9 in geöffnetem Zustand;
- Figur 13:: eine Seitenansicht des Ligaturclips der Figur 12 in teilweise geschlossenem Zustand;
- Figur 14:: eine Seitenansicht des Ligaturclips der Figur 12 in geschlossenem Zustand;
- Figur 15:: eine perspektivische Ansicht eines Trägers mit einer größeren Anzahl von Ligaturclips gemäß Figur 1;
- Figur 16:: eine perspektivische Ansicht eines weiteren bevorzugten Ausführungsbeispiels eines Ligaturclips mit T-förmiger Ausbindung der Arme in geöffnetem Zustand;
- Figur 17:: eine schematische Ansicht von zwei Hohlorganen, die mittels Ligaturclips gemäß Figur 16 miteinander verbunden sind und
- Figur 18:: eine Schnittansicht durch den Anlagebereich von zwei Hohlorganen längs Linie 18-18 in Figur 17.

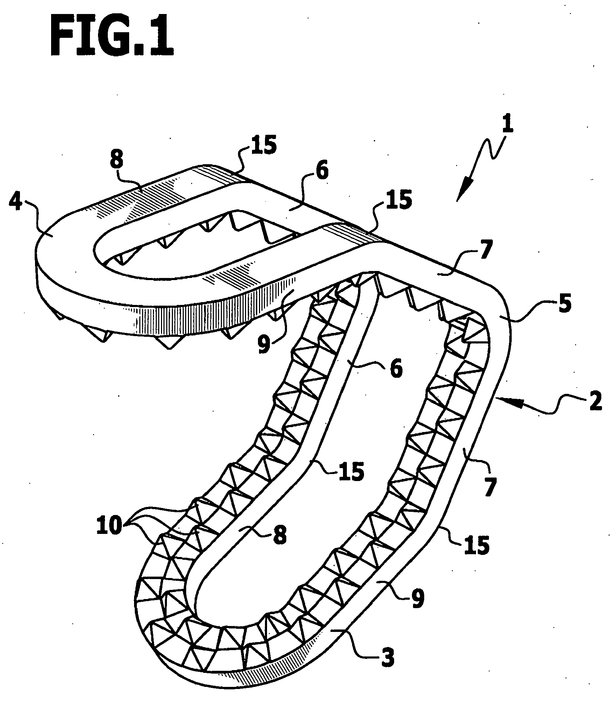

Der in den Figuren 1 bis 5 dargestellte Ligaturclip 1 besteht aus einem in sich geschlossenen streifenförmigen Band 2 aus einem dauerhaft biegbaren Material, beispielsweise aus Titan, einer Titanlegierung oder einem der zur Herstellung von derartigen Ligaturclips üblicherweise verwendeten Kunststoffe. Selbstverständlich können auch andere Materialien Verwendung finden, beispielsweise Stähle, sofern die verwendeten Materialien körperkompatibel sind.

Im dargestellten Ausführungsbeispiel hat das Band 2 einen rechteckigen Querschnitt, der über die gesamte Länge des Bandes 2 gleich bleibt und bei dem die Breite des Bandes etwa doppelt so groß ist wie die Höhe des Bandes.

Das in sich geschlossene Band 2 ist dabei so angeordnet, dass es über einen großen Teil seiner Länge zwei geradlinige, parallel im Abstand zueinander nebeneinander laufende Bereiche ausbildet, die an ihren Enden durch einen kreisbogenförmigen Verlauf des Bandes ineinander übergehen. Längs einer quer zur Längsausdehnung der geradlinigen Bereiche verlaufenden Mittellinie sind die beiden Hälften des in sich geschlossenen Bandes 2 aufeinander gefaltet, so dass sich zwei einander gegenüberstehende Arme 3, 4 ergeben, die im Bereich der genannten Mittellinie eine im Wesentlichen bogenförmig verformte Verbindungsstelle 5 ausbilden. Ausgehend von dieser Verbindungsstelle 5 verlaufen die beiden parallel nebeneinander liegenden Teile des Bandes 2 in Form von geradlinigen Abschnitten 6, 7 in einer Ebene, daran schließen sich weitere geradlinige.Abschnitte 8, 9 an, die ebenfalls in einer Ebene liegen, wobei die beiden Ebenen etwa um einen Winkel von 30° gegeneinander geneigt sind. Die beiden Abschnitte 8, 9 gehen im Bereich des freien Endes der Arme 3, 4 in Form eines sich über 180° erstreckenden Kreisbogens ineinander über.

Auf der Innenseite, die jeweils dem anderen Arm zugewandt ist, tragen beide Arme 3, 4 eine Profilierung in Form von in Richtung des anderen Armes abstehenden, spitz zulaufenden Zähnen 10, diese Profilierung erstreckt sich über die gesamte Länge des Bandes 2.

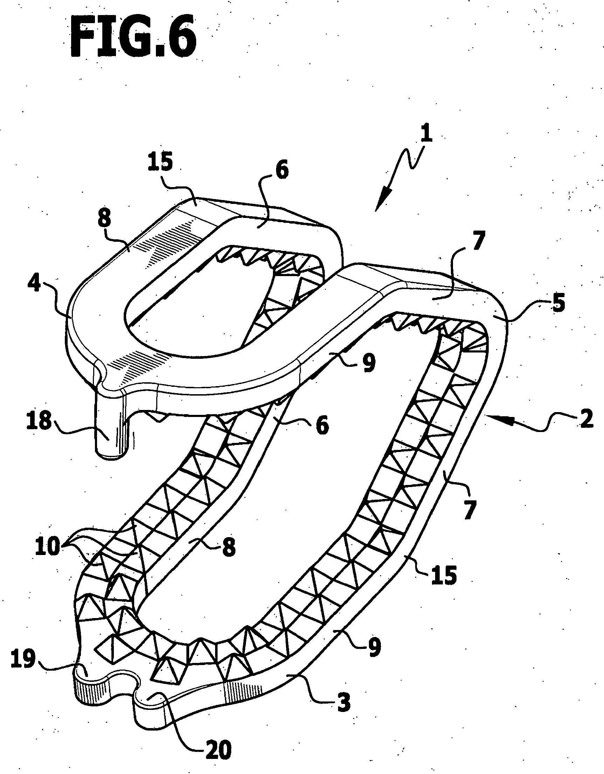

In Figur 6 ist ein Ligaturclip 1 dargestellt, der im Wesentlichen dem der Figur 1 entspricht, einander entsprechende Teile tragen daher dieselben Bezugszeichen. Im Unterschied zu dem Ligaturclip der Figur 1 tragen die beiden Arme 3, 4 des Ligaturclips 1 an ihrem freien Ende zusätzliche Vorsprünge 18, 19, 20, die so angeordnet sind, dass bei geschlossenem Ligaturclip der Vorsprung 18 an einem der beiden Arme zwischen die Vorsprünge 19, 20 am anderen Arm eintaucht und dadurch einerseits den Zwischenraum zwischen den beiden Armen 3, 4 nach vorne hin abschließt und andererseits die beiden Arme gegen seitliche Verschiebung relativ zueinander sichert.

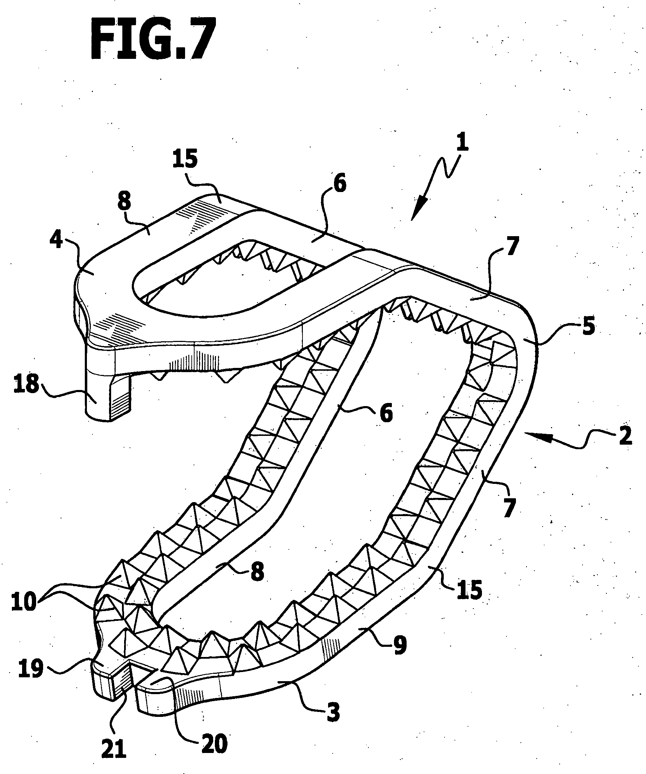

Bei dem Ligaturclip der Figur 7 ist eine sehr ähnliche Ausgestaltung gewählt, dabei sind die Vorsprünge 18, 19, 20 noch prägnanter ausgebildet, so dass der Vorsprung 18 an einem Arm in eine Ausnehmung 21 zwischen den Vorsprüngen 19, 20 am anderen Arm formschlüssig eintaucht. Eine solche Passung wird auch als Mauszahnprofilierung bezeichnet.

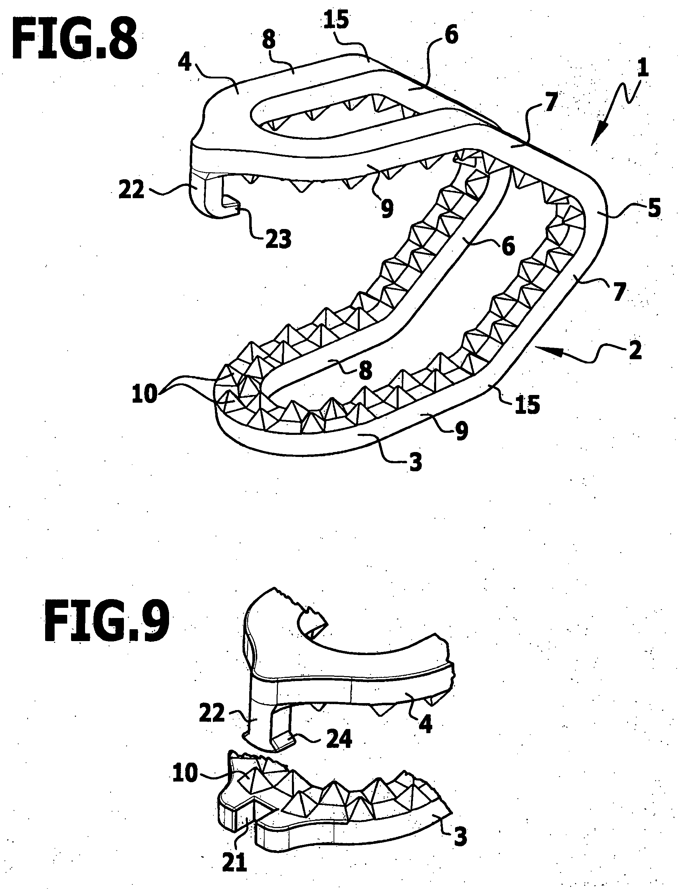

In Figur 8 ist ein ähnlicher Ligaturclip 1 gezeigt, bei dem die beiden Arme im Schließzustand miteinander verrastet werden können. Dazu trägt einer der beiden Arme an seinem freien Ende einen Rastvorsprung 22, der an seinem freien Ende seitlich abgewinkelt ist. Im geschlossenen Zustand kann die seitliche Abwinkelung 23 des Rastvorsprunges 22 am anderen Arm vorbei gleiten und diesen hintergreifen.

In Figur 9 ist eine abgewandelte Ausgestaltung einer Rastvorrichtung dargestellt, dort ist der Rastvorsprung 22 an seinem freien Ende seitlich verdickt. Der Rastvorsprung 22 greift bei geschlossenem Ligaturclip in eine Ausnehmung 21 des anderen Armes ein, und die Verdickung 24 am freien Ende des Rastvorsprungs 22 hintergreift den anderen Arm. Diese Rasteinrichtung übernimmt gleichzeitig eine ähnliche Funktion wie eine Mauszahnprofilierung, die beiden Arme werden nämlich auch noch gegen eine seitliche Verschiebung gegeneinander gesichert.

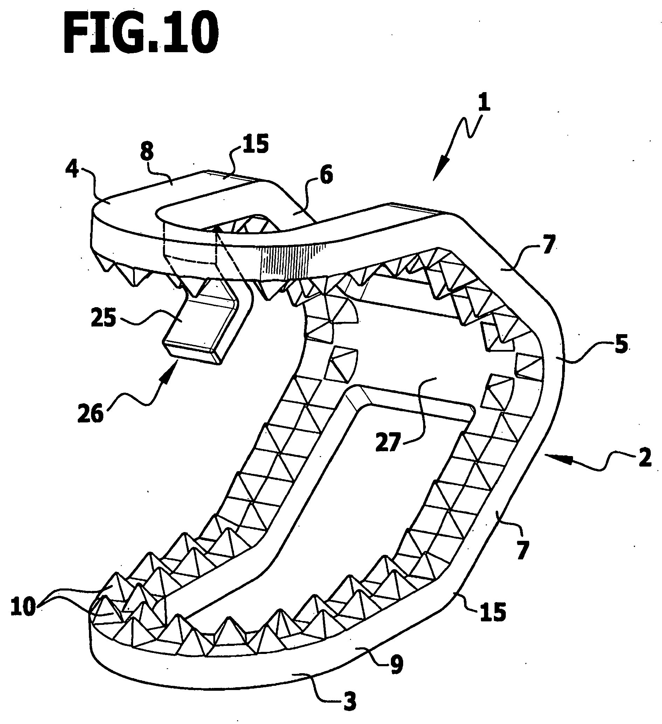

Bei dem in Figur 10 dargestellten Ligaturclip trägt einer der beiden Arme eine vom freien Ende in den Zwischenraum zwischen den nebeneinander liegenden Abschnitten ragende Zunge 25, die etwa in der Mitte ihrer Länge um fast 180° umgebogen ist und somit einen Rasthaken 26 ausbildet, der bei geschlossenem Ligaturclip in den Zwischenraum zwischen den nebeneinander liegenden Abschnitten des anderen Armes eintaucht und diesen Arm hintergreift. Auf diese Weise werden ebenfalls beide Arme im Schließzustand miteinander verrastet.

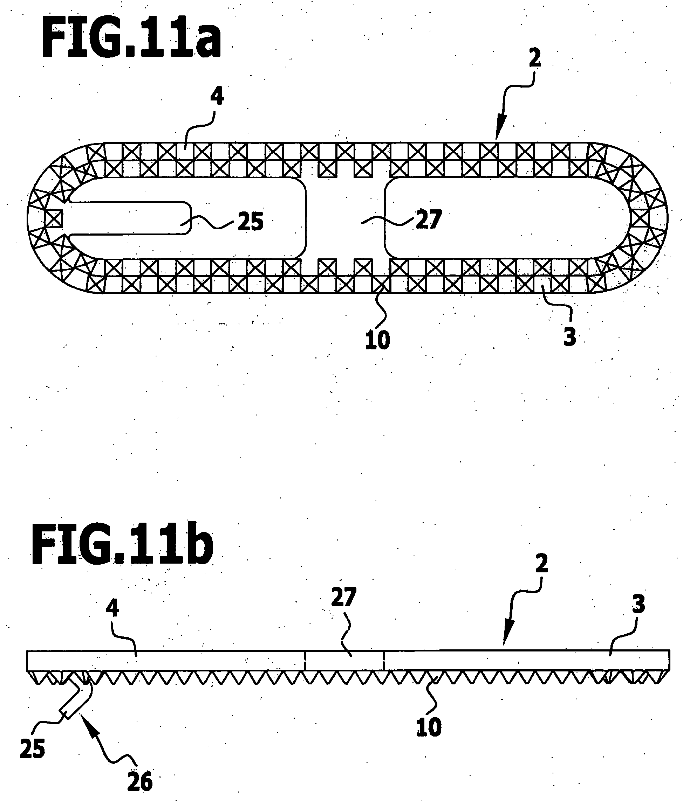

Zur Herstellung des Ligaturclips 1 wird ausgegangen von einem in sich geschlossenen Band, das in einer Ebene liegt und das mit geradlinigen Abschnitten und kreisbogenförmigen Verbindungsbereichen vorgeformt ist. In den Figuren 11a, 11b sowie 12 bis 14 wird dieser Herstellungsvorgang am Beispiel des Ligaturclips der Figur 10 dargestellt. Das Ausgangsbauteil in Form eines geschlossenen Bandes wird einmal um die Mittellinie und zum anderen im Bereich zwischen den Abschnitten 6 und 8 bzw. 7 und 9 so gebogen, dass die beiden durch diese Abschnitte jeweils aufgespannten Ebenen gegeneinander geneigt sind. Die sich unmittelbar an die Verbindungsstelle 5 anschließenden Abschnitte 6 bzw. 7 der beiden Arme 3, 4 bilden einen Öffnungswinkel von etwa 60° aus, diese Stellung wird als Offenstellung des Ligaturclips 1 bezeichnet. In dieser Stellung der Arme 3 ist der Ligaturclip 1 im Querschnitt etwa V-förmig ausgebildet und ist geeignet, mit Hilfe eines Anlegeinstrumentes 11 seitlich an ein Hohlorgan 12 angelegt zu werden, an dem er festgelegt werden soll.

Der Ligaturclip der Figuren 10, 11a und 11b weist im Bereich der Verbindungsstelle 5 einen Quersteg 27 auf, durch den die Stabilität des Ligaturclips erhöht wird, unbedingt notwendig ist dieser Quersteg allerdings nicht, die Ausführungsbeispiele der Figuren 1, 6, 7 und 8 haben keinen derartigen Quersteg.

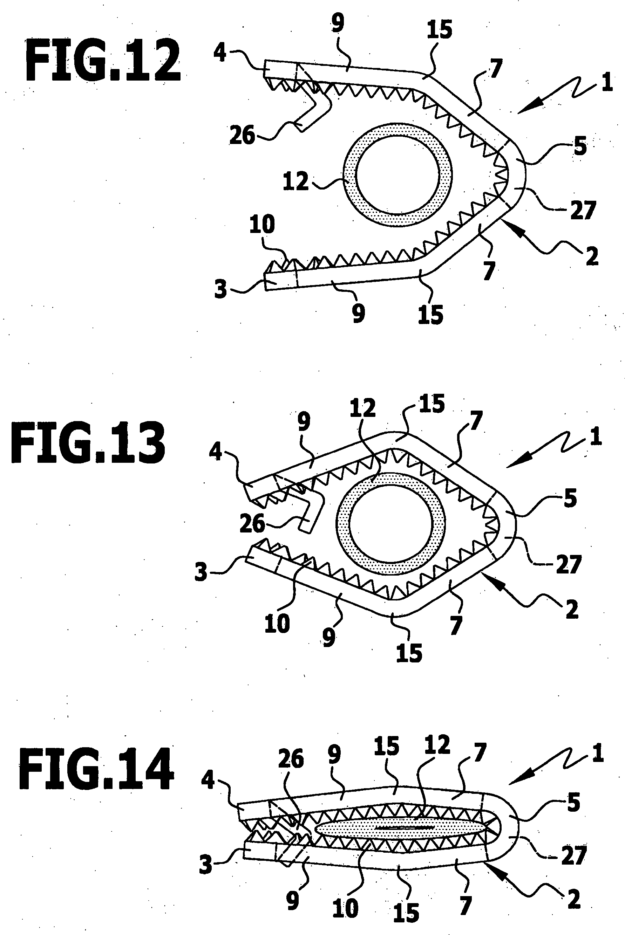

Die Figuren 12, 13 und 14 zeigen die Form des Ligaturclips beim Anlegen an ein Hohlorgan 12 während des Schließvorganges bzw. am Ende des Schließvorganges. Man erkennt deutlich, dass bei geschlossenem Ligaturclip der Rasthaken 26 den gegenüberliegenden Arm an dessen freiem Ende hintergreift und somit den Ligaturclip im Bereich der freien Enden der Arme verschließt und außerdem die beiden Arme gegen eine seitliche Verschiebung sichert.

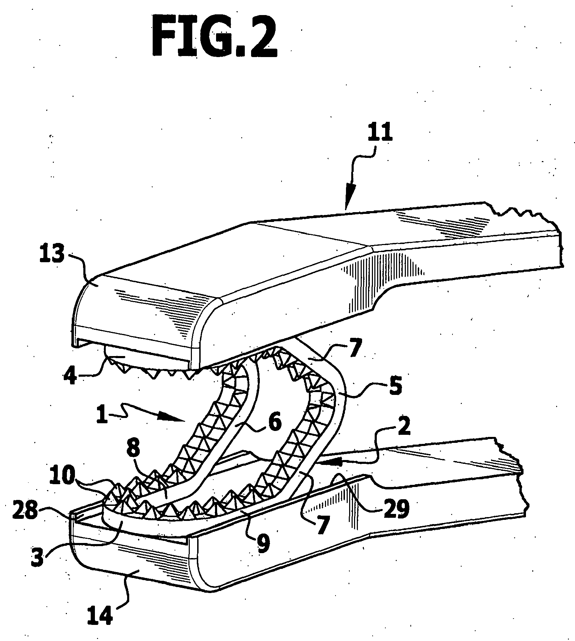

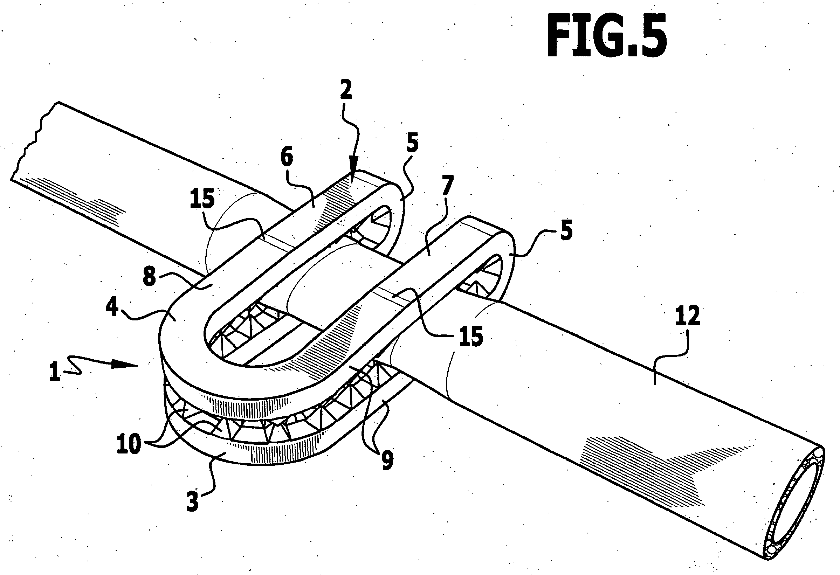

In Figur 2 ist schematisch ein Anlegeinstrument 11 mit einem eingelegten Ligaturclip 1 in Offenstellung dargestellt. Das Anlegeinstrument 11 umfasst dabei zwei an die Außenseite der beiden Arme 3, 4 anlegbare Klemmbacken 13, 14, die mittels eines in der Zeichnung nicht dargestellten Mechanismus einander angenähert werden können, so dass dadurch die beiden auf gegenüberliegenden Seiten eines Hohlorgans 12 angeordneten Arme 3, 4 gegeneinander gedrückt werden. Dabei wird der Ligaturclip 1 im Bereich der Verbindungsstelle 5 und gegebenenfalls auch im Bereich der Abwicklung 15 der Arme 3, 4 zwischen den Abschnitten 6 und 8 bzw. 7 und 9 verformt, so dass die Arme 3, 4 danach das Hohlorgan 12 fest zwischen sich einschließend aneinander anlegen und im wesentlichen gestreckt sind, wie dies in Figur 5 dargestellt ist. Die Arme 3, 4 weisen dabei zwei nebeneinander liegende Stege auf, sie sind also als Doppelclip ausgebildet und liegen in zwei nebeneinander liegenden Bereichen am Hohlorgan an, das auf diese Weise sicher von dem Ligaturclip 1 gefasst wird.

Bei dem Ausführungsbeispiel der Figur 2 sind die Klemmbacken 13, 14 des Anlegeinstrumentes 11 geringfügig breiter ausgebildet als der Ligaturclip 1, zur Zentrierung des Ligaturclips 1 tragen die Klemmbacken 13, 14 an ihren Außenkanten seitlich nach oben überstehende Leisten 28, 29, die an den Außenseiten der Abschnitte 6, 7, 8, 9 anliegen und damit eine sichere Positionierung des Ligaturclips zwischen den Klemmbacken sicherstellen.

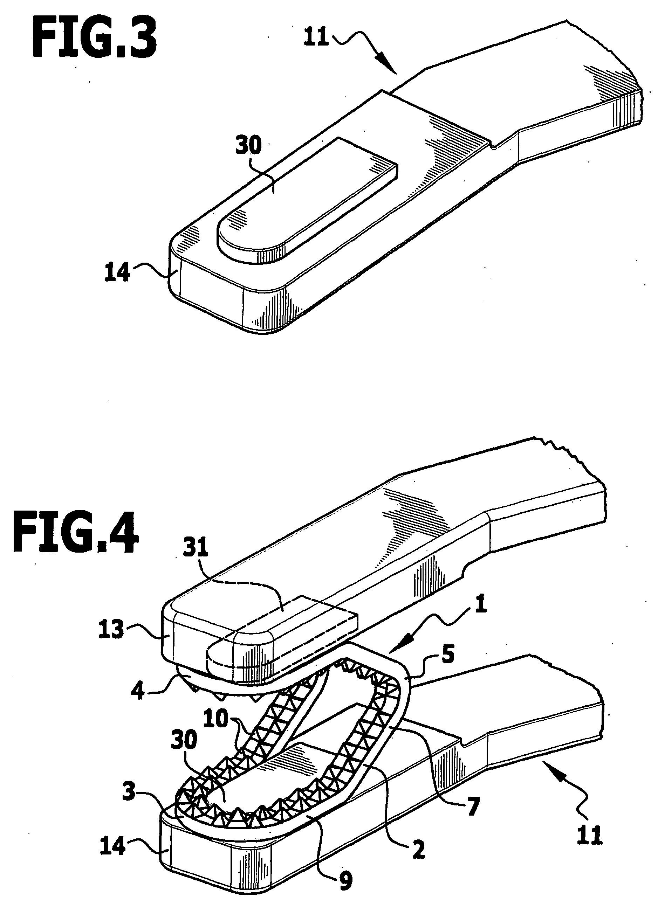

In den Figuren 3 und 4 ist ein weiteres Ausführungsbeispiel eines derartigen Anlegeinstrumentes 11 dargestellt, bei dem die Klemmbacken 13, 14 Zentriervorsprünge 30, 31 tragen, die genau in den Zwischenraum zwischen den Abschnitten 6, 7 bzw. 8, 9 der Arme 3, 4 hineinpassen, wenn die Klemmbacken 13, 14 an die Außenseiten der Arme 3, 4 angelegt sind. Auch auf diese Weise ergibt sich eine exakte und dauerhafte Positionierung des Ligaturclips zwischen den Klemmbacken 13, 14, bei diesem Ausführungsbeispiel können die Klemmbacken 13, 14 gegebenenfalls auch schmaler ausgebildet sein als die Ligaturclips 1.

In Figur 15 ist schematisch ein im Querschnitt rechteckiger Träger 16 dargestellt, auf den eine größere Anzahl von Ligaturclips 1 der in den Figuren 1 bis 3 dargestellten Art in Offenstellung aufgeschoben sind. Die Abmessungen der Trägers 16 sind dabei so gewählt, dass seine Breite der Breite des Zwischenraumes zwischen den nebeneinander verlaufenden Stegen der Arme 3, 4 entspricht, so dass die Abschnitte 6, 7, 8, 9 der Arme 3, 4 dicht am Träger 16 anliegen und die Ligaturclips 1 daher kraftschlüssig auf dem Träger 16 festlegen. Alle Ligaturclips 1 sind dabei parallel zueinander und unmittelbar nebeneinander angeordnet, so dass auf engem Raum eine größere Anzahl derartiger Ligaturclips 1 auf dem Träger 16 gelagert werden können. Mittels eines in der Zeichnung nur schematisch dargestellten Ausschubgliedes 17 können die Ligaturclips 1 auf dem Träger 16 verschoben werden, so dass auf diese Weise jeweils der vorderste Ligaturclip 1 für die Anlage freigegeben werden kann. Eine solche Freigabe kann auch durch andere Mittel erfolgen, beispielsweise durch ein Anlegeinstrument 11, welches aus dem Vorrat von Ligaturclips 1 auf dem Träger 16 jeweils nur den vordersten Ligaturclip oder nur den hintersten Ligaturclip erfasst und vom Träger abziehen kann. Wesentlich ist, dass durch die Ausgestaltung der Ligaturclips 1 mit zwei nebeneinander liegenden Stegen zwischen den Stegen ein Aufnahmeraum für den Träger gebildet wird, der ohne weitere Hilfsmittel die Lagerung der Ligaturclips 1 auf einem in diesen Aufnahmeraum eingeschobenen Träger ermöglicht.

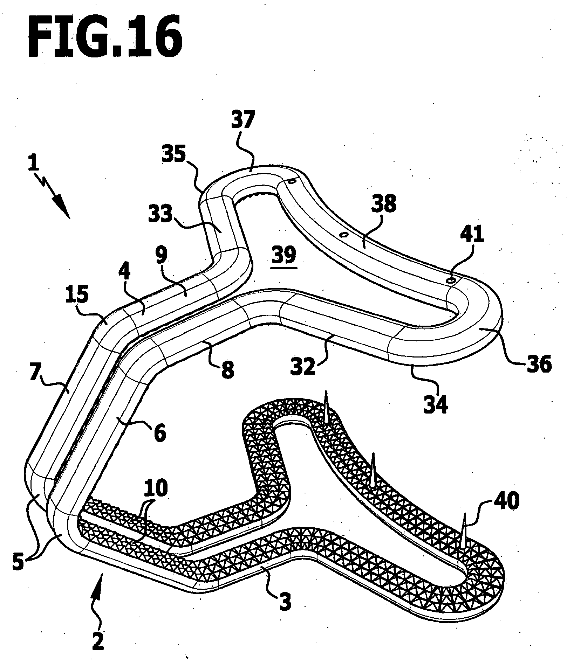

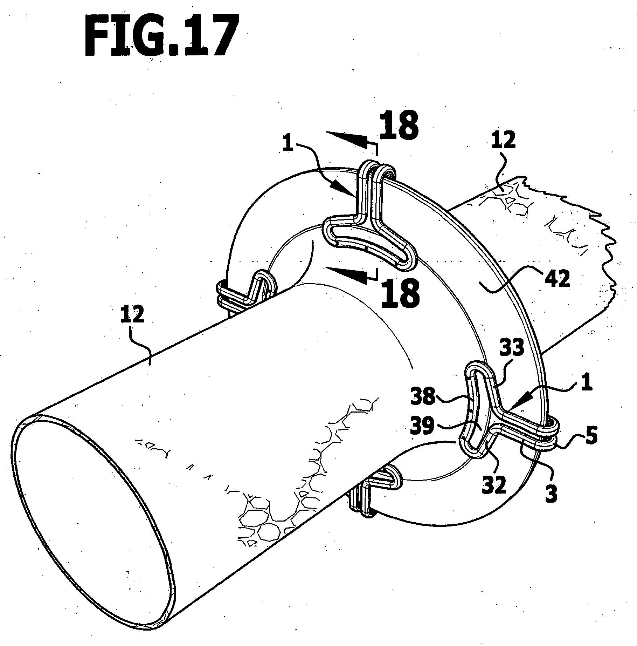

In den Figuren 16 bis 18 ist ein abgewandeltes Ausführungsbeispiel eines Ligaturclips 1 beschrieben. Dieser ist ähnlich aufgebaut wie der Ligaturclip der vorstehend beschriebenen Ausführungsbeispiele, einander entsprechende Teile tragen daher dieselben Bezugszeichen.

Bei dem Ligaturclip der Figuren 16 bis 18 gehen die nebeneinander liegenden Abschnitte 8 und 9 am freien Ende der Arme 3, 4 nicht in Form eines Bogens unmittelbar ineinander über, sondern an die freien Enden der Abschnitte 8 und 9 schließen sich nach außen und damit nach gegenüberliegenden Seiten abstehende Transversalabschnitte 32, 33 an, die in derselben Ebene verlaufen wie die nebeneinander liegenden Abschnitte 8 und 9. An ihren den Abschnitten 8 und 9 abgewandten äußeren Enden 34, 35 gehen die Transversalabschnitte 32, 33 über kreisbogenförmige, sich etwa über 180 ° erstreckende Endabschnitte 36, 37 über in einen gemeinsamen Klemmabschnitt 38, der auf der den nebeneinander liegenden Abschnitten 8, 9 abgewandten Seite der Transversalabschnitte 32, 33 im Abstand zu diesen verläuft und die beiden Endabschnitte 36, 37 miteinander verbindet. Der Klemmabschnitt 38 verläuft dabei bei dem in den Figuren 16 bis 18 dargestellten Ausführungsbeispiel zwischen den Endabschnitten 36 und 37 kreisbogenförmig, wobei die äußeren Enden 34, 35 nach außen gebogen sind, das heißt also, von den Armen 3, 4 wegweisend. Der Klemmabschnitt 38 liegt in derselben Ebene wie die Transversalabschnitte 32, 33 und die bogenförmigen Endabschnitte 36, 37, die Transversalabschnitte 32, 33, die Endabschnitte 36, 37 und der Klemmabschnitt 38 bilden eine gemeinsame Klemmfläche am freien Ende der Arme 3, 4 aus.

Bei dem Ausführungsbeispiel der Figur 16 stehen die Transversalabschnitte 32, 33 unter einem Winkel von etwa 45° von den nebeneinander liegenden Abschnitten 8, 9 ab, es kann sich dabei aber auch um einen Winkel in der Größenordnung von 90° handeln. Insbesondere können die Transversalabschnitte 32, 33 und der Klemmabschnitt 38 parallel zueinander verlaufen. In jedem Fall verbleibt zwischen den Transversalabschnitten 32, 33 und dem Klemmabschnitt 38 ein Zwischenraum 39.

Auch der Ligaturclip der Figuren 16 bis 18 ist einstückig aus einem Band 2 geformt und trägt auf seiner Innenseite Zähne 10 oder eine ähnliche Profilierung.

Außerdem sind an einem der beiden Klemmabschnitte 38 über dessen Länge verteilt mehrere stiftförmige Vorsprünge 40 angeordnet, denen jeweils im anderen Klemmabschnitt eine Bohrung 41 gegenübersteht, in die der stiftförmige Vorsprung 40 eintaucht, wenn die beiden Klemmabschnitte 38 der beiden Arme 3, 4 einander angenähert werden.

Der in den Figuren 16 bis 18 dargestellte Ligaturclip ist besonders geeignet zur Verbindung von zwei Hohlorganen 12. Diese werden an den aneinanderstoßenden Enden mit ihrem Rand 42 seitlich nach außen umgebogen, so dass ein flanschförmiger Überstand entsteht. In diesem Bereich können mehrere Ligaturclips in Umfangsrichtung nebeneinander derart angeordnet werden, dass die bogenförmigen Klemmabschnitte 38 der Außenkontur der Hohlorgane 12 folgen.

Es ist dabei insbesondere günstig, wenn Ligaturclips verwendet werden, bei denen die Krümmung der Klemmabschnitte 38 der Krümmung der Hohlorgane entspricht. Zu diesem Zweck werden in einem Satz dem Operateur Ligaturclips mit Klemmabschnitten 38 zur Verfügung gestellt, die eine unterschiedliche Krümmung aufweisen, so dass er aus diesem Satz die Ligaturclips auswählen kann, die jeweils an die Krümmung des Hohlorgans angepasst sind.

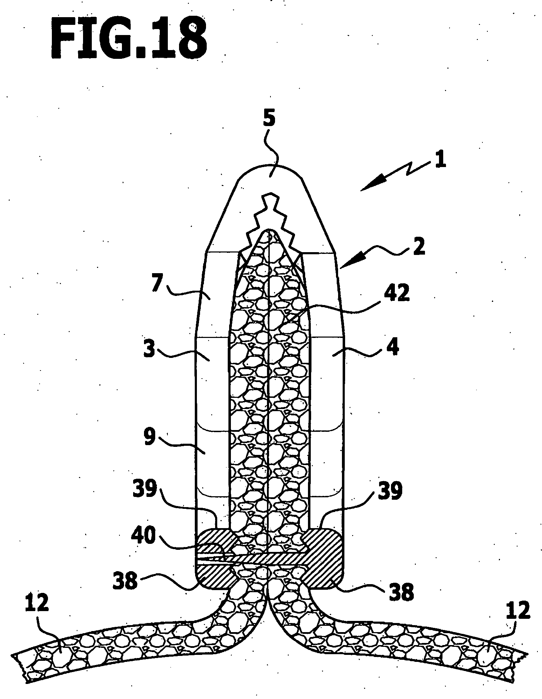

Wenn die Ligaturclips angelegt und so geschlossen sind, dass die Klemmabschnitte 38 der beiden Arme 3, 4 gegeneinander gedrückt sind, dann nehmen die Klemmabschnitte 38 und gegebenenfalls auch die Transversalabschnitte 32, 33 zwischen sich das Gewebe auf, im Beispiel der Verbindung der Hohlorgane 12 also den Rand 42, wie dies in Figur 18 dargestellt ist. Die stiftförmigen Vorsprünge 40 durchdringen dabei den Rand 42 und treten in die Bohrung 41 des gegenüberliegenden Klemmabschnittes 38 ein, so dass eine sichere Fixierung der Ligaturclips am Rand 42 sichergestellt ist. Diese sichere Fixierung wird auch dadurch unterstützt, dass das Gewebe des Randes 42 in den Zwischenraum 39 zwischen dem Klemmabschnitt 38 einerseits und den beiden Transversalabschnitten 32, 33 eintritt und dadurch eine seitliche Verschiebung des Ligaturclips gegenüber dem Rand verhindert.

## Patentansprüche

1. Satz von mehreren chirurgischen Ligaturclips (1) mit jeweils zwei Haltearmen (3, 4), die an jeweiis einem Ende über eine verformbare Verbindungsstelle (5) miteinander verbunden sind und derart gegeneinander biegbar sind, dass die Arme (3, 4) aus einer Offenstellung, in der sie einen größeren Abstand voneinander haben, in eine Schließstellung gelangen, in der die einander zugewandten Innenseiten der Arme (3, 4) dauerhaft einander angenähert sind, wobei der Ligaturclip (1) aus einem in sich geschlossenen Steg (2) besteht, der im Bereich der beiden Arme (3, 4) und der Verbindungsstelle (5) zwei nebeneinander liegende Abschnitte (6, 8; 7, 9) ausbildet, die an den der Verbindungsstelle (5) abgewandten freien Enden der Arme (3, 4) ineinander übergehen, **dadurch gekennzeichnet, dass** der Satz einen gemeinsamen Träger (16) für die Ligaturclips (1) umfasst, der die parallel zueinander und in einer Reihe nebeneinander angeordneten Ligaturclips (1) im Bereich des Zwischenraumes zwischen den nebeneinander liegenden Abschnitten (6, 8; 7, 9) durchsetzt.

2. Satz von mehreren Ligaturclips nach Anspruch 1, **dadurch gekennzeichnet, dass** die nebeneinander liegenden Abschnitte (6, 8; 7, 9) der Ligaturclips (1) zumindest stellenweise dicht an dem Träger (16) anliegen.

3. Satz von mehreren Ligaturclips nach einem der Ansprüche 1 oder 2, **dadurch gekennzeichnet, dass** die nebeneinander liegenden Abschnitte (8, 9) an ihrem der Verbindungsstelle (5) abgewandten Ende in nach gegenüberliegenden Seiten seitlich abstehende Transversalabschnitte (32, 33) einmünden.

4. Satz von mehreren Ligaturclips nach Anspruch 3, **dadurch gekennzeichnet, dass** die Transversalabschnitte (32, 33) an ihren den nebeneinander liegenden Abschnitten (8, 9) abgewandten äußeren Enden (34, 35) in einen Klemmabschnitt (38) übergehen, der im Abstand von den Transversalabschnitten (32, 33) auf deren den nebeneinander liegenden Abschnitten (8, 9) abgewandter Seite verläuft.

5. Satz von mehreren Ligaturclips nach Anspruch 4, **dadurch gekennzeichnet, dass** der Klemmabschnitt (38) im wesentlichen parallel zu den Transversalabschnitten (32, 33) verläuft.

6. Satz von mehreren Ligaturclips nach Anspruch 4 oder 5, **dadurch gekennzeichnet, dass** die Transversalabschnitte (32, 33) an den äußeren Enden (34, 35) bogenförmig in den Klemmabschnitt (38) übergehen.

7. Satz von mehreren Ligaturclips nach Anspruch 6, **dadurch gekennzeichnet, dass** die Transversalabschnitte (32, 33) an den äußeren Enden (34, 35) kreisbogenförmig in den Klemmabschnitt (38) übergehen.

8. Satz von mehreren Ligaturclips nach einem der Ansprüche 3 bis 7, **dadurch gekennzeichnet, dass** die Transversalabschnitte (32, 33) und gegebenenfalls der Klemmabschnitt (38) in derselben Ebene liegen wie die an die Transversalabschnitte (32, 33) anschließenden nebeneinander liegenden Abschnitte (8, 9).

9. Satz von mehreren Ligaturclips nach einem der Ansprüche 4 bis 8, **dadurch gekennzeichnet, dass** der Klemmabschnitt (38) bogenförmig verläuft.

10. Satz von mehreren Ligaturclips nach Anspruch 9, **dadurch gekennzeichnet, dass** der Klemmabschnitt (38) kreisbogenförmig verläuft.

11. Satz von mehreren Ligaturclips nach einem der Ansprüche 9 oder 10, **dadurch gekennzeichnet, dass** der Klemmabschnitt (38) von den nebeneinander liegenden Abschnitten (8, 9) wegweisend gebogen ist.

12. Satz von mehreren Ligaturclips nach einem der Ansprüche 4 bis 11, **dadurch gekennzeichnet, dass** mindestens ein Klemmabschnitt (38) einen Vorsprung (40) trägt, der bei aneinander liegenden Klemmabschnitten (38) der beiden Arme (3, 4) in eine Ausnehmung (41) des anderen Klemmabschnitt (38) eintaucht.

13. Satz von mehreren Ligaturclips nach Anspruch 12, **dadurch gekennzeichnet, dass** der Vorsprung (40) ein Stift ist.

14. Satz von mehreren Ligaturclips nach Anspruch 12 oder 13, **dadurch gekennzeichnet, dass** an dem Klemmabschnitt (38) eines Armes (3, 4) über dessen Längsrichtung verteilt mehrere Vorsprünge (40) und an dem Klemmabschnitt (38) des anderen Armes entsprechende Ausnehmungen (41) angeordnet sind.

## Claims

1. Set of a plurality of surgical ligature clips (1), each comprising two retaining arms (3, 4), which are connected to one another in each case at one end by a deformable connection point (5) and bendable in such a way towards one another that the arms (3, 4) move from an open position, in which they are spaced further apart from one another, into a closed position, in which the mutually opposing inner sides of the arms (3, 4) are brought permanently closer to one another, the ligature clip (1) consisting of an endless web member (2), which in the region of the two arms (3, 4) and the connection point (5) forms two juxtaposed portions (6, 8; 7, 9) that merge into one another at the free ends of the arms (3, 4) remote from the connection point (5), **characterized in that** the set comprises a common carrier (16) for the ligature clips (1), that passes through the ligature clips (1), which are disposed parallel and in a row adjacent to one another, in the region of the space between the juxtaposed portions (6, 8; 7, 9).

2. Set of a plurality of ligature clips according to claim 1, **characterized in that** the juxtaposed portions (6, 8; 7, 9) of the ligature clips (1) lie at least in places closely adjacent to the carrier (16).

3. Set of a plurality of ligature clips according to one of claims 1 or 2, **characterized in that** the juxtaposed portions (8, 9) at their end remote from the connection point (5) open into transverse portions (32, 33) that project laterally in opposite directions.

4. Set of a plurality of ligature clips according to claim 3, **characterized in that** the transverse portions (32, 33) at their outer ends (34, 35) remote from the juxtaposed portions (8, 9) merge into a clamping portion (38) that extends at a spacing from the transverse portions (32, 33) at the side thereof remote from the juxtaposed portions (8, 9).

5. Set of a plurality of ligature clips according to claim 4, **characterized in that** the clamping portion (38) extends substantially parallel to the transverse portions (32, 33).

6. Set of a plurality of ligature clips according to claim 4 or 5, **characterized in that** the transverse portions (32, 33) at the outer ends (34, 35) merge in an arcuate manner into the clamping portion (38).

7. Set of a plurality of ligature clips according to claim 6, **characterized in that** the transverse portions (32, 33) at the outer ends (34, 35) merge in a circular arcuate manner into the clamping portion (38).

8. Set of a plurality of ligature clips according to one of claims 3 to 7, **characterized in that** the transverse portions (32, 33) and optionally the clamping portion (38) lie in the same plane as the juxtaposed portions (8, 9) adjoining the transverse portions (32, 33).

9. Set of a plurality of ligature clips according to one of claims 4 to 8, **characterized in that** the clamping portion (38) extends in an arcuate manner.

10. Set of a plurality of ligature clips according to claim 9, **characterized in that** the clamping portion (38) extends in a circular arcuate manner.

11. Set of a plurality of ligature clips according to one of claims 9 or 10, **characterized in that** the clamping portion (38) is bent in a direction away from the juxtaposed portions (8, 9).

12. Set of a plurality of ligature clips according to one of claims 4 to 11, **characterized in that** at least one clamping portion (38) carries a projection (40) that, when the clamping portions (38) of the two arms (3, 4) lie adjacent to one another, engages into a recess (41) of the other clamping portion (38).

13. Set of a plurality of ligature clips according to claim 12, **characterized in that** the projection (40) is a pin.

14. Set of a plurality of ligature clips according to claim 12 or 13, **characterized in that** a plurality of projections (40) are disposed on, and distributed over the longitudinal direction of, the clamping portion (38) of one arm (3, 4) and corresponding recesses (41) are disposed on the clamping portion (38) of the other arm.

## Revendications

1. Jeu de plusieurs pinces de ligature chirurgicales (1) comprenant chacune deux bras de maintien (3, 4) qui sont reliés l'un à l'autre à une extrémité respective via un emplacement de liaison déformable (5), et qui peuvent être cintrés l'un vers l'autre de telle façon que les bras (3, 4), en partant d'une position ouverte dans laquelle ils présentent une distance plus grande l'un par rapport à l'autre, parviennent dans une position fermée dans laquelle les faces intérieures, tournées l'une vers l'autre, des bras (3, 4), sont rapprochées durablement l'une de l'autre, ladite pince de ligature (1) étant formée d'une barrette (2) refermée sur elle-même, qui, dans la région des deux bras (3, 4) et de l'emplacement de liaison (5), forme deux portions (6, 8; 7, 9) situées l'une à côté de l'autre, qui se raccordent l'une à l'autre au niveau des extrémités libres éloignées de l'emplacement de liaison (5) des bras (3, 4),
**caractérisé en ce que** le jeu comporte un support commun (16) pour les pinces de ligature (1), qui traverse les pinces de ligature (1) agencées côte à côte parallèlement les unes aux autres en une rangée, dans la région de l'espace intermédiaire entre les portions (6, 8; 7, 9) situées l'une à côté de l'autre.

2. Jeu de plusieurs pinces de ligature selon la revendication 1, **caractérisé en ce que** les portions (6, 8; 7, 9) situées l'une à côté de l'autre, des pinces de ligature (1), s'appliquent, au moins par endroits, étroitement contre le support (16).

3. Jeu de plusieurs pinces de ligature selon l'une des revendications 1 ou 2, **caractérisé en ce que** les portions (8, 9) situées l'une à côté de l'autre débouchent, au niveau de leur extrémité éloignée de l'emplacement de liaison (5), dans des portions transversales (32, 33), qui font saillie latéralement vers des côtés opposés.

4. Jeu de plusieurs pinces de ligature selon la revendication 3, **caractérisé en ce que** le portions transversales (32, 33) se raccordent, à leurs extrémités extérieures (34, 35) éloignées des portions (8, 9) situées l'une à côté de l'autre, à une portion de serrage (38), qui s'étend à distance des portions transversales (32, 33), sur leur côté opposé à celui dirigé vers les portions (8, 9) situées l'une à côté de l'autre.

5. Jeu de plusieurs pinces de ligature selon la revendication 4, **caractérisé en ce que** la portion de serrage (38) s'étend sensiblement de manière parallèle aux portions transversales (32, 33).

6. Jeu de plusieurs pinces de ligature selon la revendication 4 ou la revendication 5, **caractérisé en ce que** les portions transversales (32, 33) se raccordent en forme d'arc à la portion de serrage (38), au niveau de leurs extrémités extérieures (34, 35).

7. Jeu de plusieurs pinces de ligature selon la revendication 6, **caractérisé en ce que** les portions transversales (32, 33) se raccordent en forme d'arc circulaire à la portion de serrage (38), au niveau de leurs extrémités extérieures (34, 35).

8. Jeu de plusieurs pinces de ligature selon l'une des revendications 3 à 7, **caractérisé en ce que** les portions transversales (32, 33) et, le cas échéant, la portion de serrage (38) sont situées dans le même plan que les portions (8, 9) situées l'une à côté de l'autre, qui succèdent aux portions transversales (32, 33).

9. Jeu de plusieurs pinces de ligature selon l'une des revendications 4 à 8, **caractérisé en ce que** la portion de serrage (38) s'étend en forme d'arc.

10. Jeu de plusieurs pinces de ligature selon la revendication 9, **caractérisé en ce que** la portion de serrage (38) s'étend en forme d'arc circulaire.

11. Jeu de plusieurs pinces de ligature selon l'une des revendications 9 ou 10, **caractérisé en ce que** la portion de serrage (38) est cintrée en s'éloignant des portions (8, 9) situées l'une à côté de l'autre.

12. Jeu de plusieurs pinces de ligature selon l'une des revendications 4 à 11, **caractérisé en ce qu'**au moins une portion de serrage (38) porte une saillie ou protubérance (40), qui, lorsque les portions de serrage (38) des deux bras (3, 4) sont appliquées l'une contre l'autre, plonge dans un évidement (41) de l'autre portion de serrage (38).

13. Jeu de plusieurs pinces de ligature selon la revendication 12, **caractérisé en ce que** la saillie ou protubérance (40) est une tige ou une broche.

14. Jeu de plusieurs pinces de ligature selon la revendication 12 ou la revendication 13, **caractérisé en ce que** plusieurs saillies ou protubérances (40) sont agencées sur la portion de serrage (38) d'un des bras (3, 4) de manière répartie le long de sa direction longitudinale, et des évidements correspondants (41) sont agencés sur la portion de serrage (38) de l'autre bras.
